# EUROPEAN PATENT APPLICATION

(11) **EP 1 900 820 A1**
(43) Date of publication of application: **19.03.2008**
(21) Application number: 06019155.8
(22) Date of filing: 13.09.2006
(51) Int. Cl.: C12N 15/87

(54) **Nuclear transport agent and method for producing said agent**

(71) Applicant: Amaxa AG, 50829 Köln (DE)
(72) Inventor: Siebenkotten, Gregor, Dr., 50226 Frechen-Königsdorf (DE)
(74) Representative: Remus, Alvaro Johannes

(57) **Abstract**

The present invention relates to a transport agent for transporting nucleic acids into eukaryotic cells and a method for producing said agent. The invention further concerns methods for transporting nucleic acids into eukaryotic cells using the transport agent according to the invention. The present invention provides an alternative transport agent and a method which are effective to allow an efficient transport of nucleic acids into eukaryotic cells. The transport agent comprises a complex forming moiety that is capable of forming complexes with at least one nucleic acid molecule and condensing said nucleic acid molecule, and at least one nuclear localization moiety comprising at least one nuclear localization signal and having an approximately neutral net charge.

SEQUENCE LISTING

<110>  amaxa GmbH

<120>  Transport agent and method for producing said agent

<130>  A02 169 EP

<160>  16

<170>  PatentIn version 3.3

<210>  1
<211>  14
<212>  PRT
<213>  Artificial

<220>
<223>  SV40 large T-antigen core NLS (PKKKRKV) with arbitrarily chosen
       flanking amino acids (glycine and serine)

<400>  1

Gly Ser Gly Ser Pro Lys Lys Lys Arg Lys Val Gly Ser Gly
1               5                   10

<210>  2
<211>  14
<212>  PRT
<213>  Artificial

<220>
<223>  SV40 large T-antigen core NLS (PPKKKRKV) with EED N-terminal
       flanking region of the NLS form Polyoma virus VP2-protein

<400>  2

Glu Glu Asp Thr Pro Pro Lys Lys Lys Arg Lys Val Glu Asp
1               5                   10

<210>  3
<211>  22
<212>  PRT
<213>  Artificial

<220>
<223>  NLS of the Influenza virus nuclear protein

<400>  3

Met Ala Ser Gln Gly Thr Lys Arg Ser Tyr Glu Gln Met Glu Thr Asp
1               5                   10                  15

Gly Glu Arg Gln Tyr Cys
            20

<210>  4
<211>  21
<212>  PRT
<213>  Artificial

<220>
<223>  Mutated part of the SV40 large T-antigen bearing the core NLS

<400>  4

Gly Lys Pro Thr Ala Asp Asp Gln His Ser Thr Pro Pro Lys Lys Lys
1               5                   10                  15

Arg Lys Val Glu Asp
            20

<210>  5
<211>  27
<212>  PRT
<213>  Artificial

<220>
<223>  Natural part of the SV40 large T-antigen bearing  the core NLS

<400>  5

Gly Lys Pro Ser Ser Asp Asp Glu Ala Thr Ala Asp Ser Gln His Ser
1               5                   10                  15

Thr Pro Pro Lys Lys Lys Arg Lys Val Glu Asp
            20                  25

<210>  6
<211>  14
<212>  PRT
<213>  Artificial

<220>
<223>  4-fold positively charged

<400>  6

Glu Ser Gly Ser Pro Lys Lys Lys Arg Lys Val Gly Ser Gly
1               5                   10

<210>  7
<211>  14
<212>  PRT
<213>  Artificial

<220>
<223>  4-fold positively charged

<400>  7

Gly Ser Gly Ser Pro Lys Lys Lys Arg Lys Val Asp Ser Gly
1               5                   10

<210>  8
<211>  14
<212>  PRT
<213>  Artificial

<220>
<223>  3-fold positively charged

<400>  8

Glu Ser Gly Ser Pro Lys Lys Lys Arg Lys Val Asp Ser Gly
1               5                   10

<210>  9
<211>  14
<212>  PRT
<213>  Artificial

<220>
<223>  3-fold positively charged

<400>  9

Glu Asp Gly Ser Pro Lys Lys Lys Arg Lys Val Gly Ser Gly
1               5                   10


<210>  10
<211>  14
<212>  PRT
<213>  Artificial

<220>
<223>  3-fold positively charged

<400>  10

Gly Ser Gly Glu Pro Lys Lys Lys Arg Lys Val Asp Ser Gly
1               5                   10


<210>  11
<211>  14
<212>  PRT
<213>  Artificial

<220>
<223>  3-fold positively charged

<400>  11

Gly Ser Asp Thr Pro Pro Lys Lys Lys Arg Lys Val Glu Ser
1               5                   10


<210>  12
<211>  14
<212>  PRT
<213>  Artificial

<220>
<223>  3-fold positively charged

<400>  12

Gly Ser Gly Thr Pro Pro Lys Lys Lys Arg Lys Val Glu Asp
1               5                   10


<210>  13
<211>  14
<212>  PRT
<213>  Artificial

<220>
<223>  4-fold positively charged

<400>  13

Gly Glu Ser Thr Pro Pro Lys Lys Lys Arg Lys Val Gly Ser
1               5                   10

<210>  14
<211>  14
<212>  PRT
<213>  Artificial

<220>
<223>  4-fold positively charged

<400>  14

Gly Ser Gly Thr Pro Pro Lys Lys Lys Arg Lys Val Glu Ser
1               5                   10

<210>  15
<211>  21
<212>  PRT
<213>  Artificial

<220>
<223>  3-fold positively charged

<400>  15

Gly Lys Pro Thr Ala Gly Asp Gln His Ser Thr Pro Pro Lys Lys Lys
1               5                   10                  15

Arg Lys Val Glu Asp
            20

<210>  16
<211>  21
<212>  PRT
<213>  Artificial

<220>
<223>  4-fold positively charged

<400>  16

Gly Lys Pro Thr Ala Asp Asp Gln His Ser Thr Pro Pro Lys Lys Lys
1               5                   10                  15

Arg Lys Val Ser Gly
            20

## Description

### Field of the invention

The present invention relates to a transport agent for transporting nucleic acids into eukaryotic cells and a method for producing said agent. The invention further concerns methods for transporting nucleic acids into eukaryotic cells using the transport agent according to the invention.

### Background of the invention

Genetic material is active in the nucleus. The transport there can occur coincidentally during cell division when the nuclear envelope temporarily disintegrates in the course of mitosis or it has to be carried out actively. Thus, the active transport into the nucleus of living cells is necessary for the transfer of genetic material into all of those cells that do not divide in the period before the intended expression of the genetic material. A nuclear transport system for nucleic acids is very important because it is effective to allow the efficient transfer of DNA into those cells that divide rarely or not at all. Most primary cells belong to this group. Primary cells are of the highest scientific interest for two reasons. First, these cells that have freshly been isolated from the organism much better reflect the functional state of the cell type than cell lines derived from them. Second, they are the target cells for gene therapy. In addition, a nuclear transport system increases the efficiency of DNA transfer into established cell lines by enabling those cells to express transferred genetic material that have not divided in the period of time between start of transfer and analysis.

The bilayer membrane that envelops the nucleus includes pores. Small molecules can traverse these pores by diffusion. In order to be able to enter the nucleus, proteins larger than about 50 kDa need a nuclear localization signal (NLS) that has to be recognized by the transport machinery. An NLS is a signal peptide that mediates transfer of a cargo, which may be another peptide, from the cytoplasm into the nucleus of a cell. Typically, a functional signal consists of four to eight amino acids, is rich in positive amino acids Arginine and Lysine and contains Prolines. It is strongly conserved in evolution so that mammalian NLS also function in yeast. A classical NLS is, for example, the SV40 large T-antigen core sequence PKKKRKV. Heterologous NLS may also be used as a tool to transport target molecules into the nucleus. For this purpose, NLS can be incorporated into the sequences of cytoplasmic proteins at relatively random positions or NLS peptides can be coupled chemically to proteins or even gold particles (reviewed in Görlich, 1998). An overview of NLS is given by T. Boulikas (1993).

Nagasaki et al. (2003) demonstrate that nuclear localization of plasmid DNA cannot be achieved by injecting conjugates of DNA and classical cationic NLS into the cytoplasm of cells. On the other hand, several artificial systems have been described that are supposed to increase transfection efficiency with the help of peptides or proteins containing nuclear localization signals.

Subramanian et al. (1999) disclose a conjugate of a non-classical NLS and a cationic peptide scaffold derived from a scrambled sequence of the SV40 T-antigen consensus NLS in order to improve the final step of nuclear import with lipofection of non-dividing cells.

US patent 5,670,347 discloses a peptide that consists of a DNA-binding basic region, a flexible hinge region and an NLS. As DNA binding is achieved by the amino acids' positive charges also in this case, the reagent forms complexes with the DNA that are meant to serve for the transport across the cellular membrane at the same time. It is not evident why the NLS sequence should not participate in the binding of DNA, so that the actual signal for the nuclear transport proteins is likely to be masked by the DNA as long as the peptide is linked to it. Moreover, the complexes generated may become very large, cf. amongst others Emi et al. (1997), what would impair transport through the nuclear pores, cf. amongst others Yoneda et al. (1987, 1992).

WO 01/38547 A2 discloses polypeptides for transfer of molecules into eukaryotic cells that comprise at least two peptide monomers which each include a nuclear localization signal or a protein transduction domain. The nuclear localization signals used are classical NLS that naturally occur in proteins.

US 6 521 456 B1 corresponding to WO 00/40742 A1 discloses a nuclear transport agent comprising a DNA-binding part that binds specifically to DNA and an extended nuclear localization signal that has a substantially neutral net charge.

WO 02/055721 A2 discloses a modular transfection system comprising a protein that is capable of forming nucleoprotein filaments (NPFs) if loaded onto a nucleic acid to be transfected. The NPF-forming protein may be modified with a nuclear localization signal in order to improve transport of the nucleic acid into the nucleus of eukaryotic cells.

### Summary of the invention

The problem underlying the present invention is to provide an alternative transport agent and method which are effective to allow an efficient transport of nucleic acids into eukaryotic cells.

The problem is solved by a transport agent that comprises a complex forming moiety that is capable of forming complexes with at least one nucleic acid molecule and condensing said nucleic acid molecule, and at least one nuclear localization moiety comprising at least one nuclear localization signal (NLS) and having an approximately neutral net charge. The combination of a moiety that binds and condenses nucleic acid molecules with a moiety that comprises an NLS but has a substantially neutral net charge leads to an effective transport agent which allows for a highly efficient transfer of molecules into eukaryotic cells. Compacting or condensing the nucleic acid to be transported facilitates the transfer through the cell membrane as well as through the nuclear pores. The compact volume, shape and size dimensions of the complex of the complex forming moiety and the nucleic acid to be transported are thus a crucial feature of the transport agent according to the invention. Additionally, the NLS sequence that serves for the transfer of said complex into the nucleus of the cell does not mediate non-specific binding to the nucleic acid since the nuclear localization moiety has an approximately neutral net charge.

So-called non-classical NLS, as for example the NLS from influenza virus nucleoprotein (Wang et al., 1997, Neumann et al., 1997), may be used in the nuclear localization moiety. Non-classical NLS do not possess a large excess of positive charges or do not reach the nucleus via the classic route of transport. However, non-classical NLS may be less efficient in mediating nuclear transport and have by far not as extensively been shown to transport cargos other than proteins. The only case in which nuclear transport of DNA with the help of a non-classical NLS is discussed is the publication by Subramanian et al. (1999) cited above. A 38 amino acid non-classical NLS (the M9 sequence of heterogeneous nuclear ribonucleoprotein) is coupled to a scrambled version of classical NLS of the Simian Virus large T-antigen. This peptide complexed with DNA leads to an enhancment of expression when transfected together with cationic liposomes as compared to the cationic liposomes plus DNA alone. This enhancement is mostly not an effect of complexing the M9 sequence to DNA since said peptide enhances the transfection efficiency only by a factor of 1.1 - 1.3 as compared to a mixture of DNA and peptides of both sequences added separately to DNA. The M9 sequence alone is not likely to bind DNA since it has only two positively charged aminoacids in 39 amino acids. In addition the synthesis of a 39 amino acid peptide is more complicated and expensive than e.g. a 14 amino acid peptide (NLS-2).

The nuclear localization signal as it is used in the transport agent according to the present invention is preferably a "classical" nuclear localization signal. Classical NLS sequences have a positive net charge as the positive charges of the basic amino acids in the core sequence are an essential part of most native nuclear localization signals. But it is a drawback of classical nuclear localization signals that they tend toward binding of nucleic acids via their positive charges so that these charges are masked and their function as signals for the nuclear transport machinery is significantly impaired. According to the invention, this drawback of classical NLS is eliminated by neutralizing the positive charges of the NLS so as to obtain a nuclear localization moiety that has a substantially neutral net charge. The neutral nuclear localization moiety including the classical NLS does not bind nucleic acids non-specifically and therefore transfer of the nucleic acid to be transported into the nucleus can be optimized.

The NLS of the nuclear localization moiety may be a bipartite nuclear localization signal, i.e. a signal that consists of two separate cationic amino acid sequences.

In a preferred embodiment, the nuclear localization signal comprises a core sequence that is capable of mediating transport of said nucleic acid into the nucleus of a cell, said core sequence having a positive net charge. "Core sequence" in this context means an amino acid sequence that corresponds to a classical NLS sequence and that is sufficient to mediate nuclear transport.

In an advantageous embodiment of the invention, the nuclear localization moiety further comprises at least one charge carrier that has a negative net charge. The charge carrier may be a short peptide or any other charged molecule. In a preferred embodiment, the charge carrier comprises at least one negatively charged amino acid.

Preferably, the approximately neutral net charge of the nuclear localization moiety is achieved by at least partially neutralizing the positive net charge of the core sequence with one or more charge carrier(s). The charge carriers, e.g. acidic amino acids, are introduced into the nuclear localization moiety by coupling them to the core sequence of the NLS, i.e. the charge carriers flank the core sequence. The flanking charge carrier(s) is (are) coupled directly to the core sequence or spaced in intervals of one or more residues, for example in the case of amino acids by neutral amino acids. In any case, the charge carrier(s) should be located within the nuclear localization moiety in vicinity of the positive charges of the core sequence in order to ensure efficient neutralization of these charges. The flanking charge carrier(s) can be coupled to the N-terminus or C-terminus of the NLS or core sequence. In the case of bipartite NLS the flanking charge carrier(s) may also be introduced between the two parts of the signal sequences.

In order to effectively avoid non-specific binding of the nuclear localization moiety to nucleic acids the nuclear localization moiety should at most have a 3-fold positive net charge as NLS sequences having more than three positive amino acids actually bind to DNA.

In a preferred embodiment, the nuclear localization signal comprises a native amino acid sequence that has a positive net charge as present in all naturally occurring classical nuclear localization signals.

With the transport agent according to the invention resting or slowly dividing primary cells can be efficiently transfected to a percentage that allows subsequent analysis. Most cells freshly isolated from the body of an animal or human (primary cells) do not divide at all or so rarely that DNA, after it has been transported across the cellular membrane successfully, is inactivated before it reaches the nucleus and can be expressed. So far this has led to most primary cells being untransfectable unless they were artificially stimulated to proliferate in culture. It is one of the unavoidable consequences that these cells then deviate from their original state. A method for the transfection of primary cells permits the analysis of genetic material under the original conditions of a body cell. This is of paramount importance for the investigation of genetic mechanisms and the study of processes inside of a body cell. Provision of the transport agent according to the invention is also an essential step toward a completely artificial gene transfer system for gene therapy. Such a gene transfer system must possess three functional components: one component for the passage of DNA through the cellular membrane, for which cationic lipids and cationic polymers have proved to be relatively suitable. It has to contain a second component for the transfer of the DNA into the nucleus of the (usually non-dividing) target cells and a third component that mediates the integration of the DNA into the genome. According to the present invention an efficient transport agent and method is described that can serve at least as the second component and to a lesser extend also as the first. A completely artificial gene transfer vehicle that can be employed in gene therapy will in all likelihood be easier and less expensive to produce and easier to handle than the viral systems currently applied, and it is not subject to the immanent risks of these systems. Gene therapeutic approaches have been suggested, for example, for the treatment of cancer, AIDS and various hereditary diseases and will play a significant role in medicine. The transport agent according to the present invention also increases the transfection efficiency in cultured cells. It does so by making those cells accessible for the uptake of DNA that do not divide in the time slot between passage of the DNA through the cellular membrane and analysis. This is important because even for many established cell lines an increase in transfection efficiency would facilitate the analysis and help to lower costs due to the reduced amount of cell material required. Of course, this is also true for all stages in between primary cells and established cell lines.

Preferably, the complex forming moiety comprises a basic peptide, a basic polypeptide or a basic protein and/or a cationic peptide, a cationic lipid or a non-lipid cationic polymer. As non-lipid cationic polymer polyethylenimine (PEI) may be used. In an advantageous embodiment of the invention, the complex forming moiety comprises Polylysine, Polyarginine, Polyornithine or Polyhistidine and/or any polypeptide composed of any mixture of the amino acids Lysine, Arginine, Ornithine and Histidine. Polypeptides comprising both moieties are easy to produce in a one step peptide synthesis.

Alternatively, the complex forming moiety may comprise a protein belonging to the High Mobility Group (HMG) protein or Histone family. HMG proteins and histones are basic proteins capable of binding and condensing DNA (WO 97/12051 A) and are thus a suitable component of the nuclear localization moiety. Human variants of these proteins may be used as potentially non-immunogenic first moieties of the transport agent if used in conjunction with gene therapy.

In an advantageous embodiment of the invention, the nuclear localization moiety comprises at least one amino acid sequence according to SEQ ID NO:2, SEQ ID NO:3, SEQ ID NO:4, SEQ ID NO:5, SEQ ID NO:8, SEQ ID NO:9, SEQ ID NO:10, SEQ ID NO:11, SEQ ID NO:12 and/or SEQ ID NO:15.

The transport agent according to the invention may additionally comprise two or more nuclear localization moieties. These additional moieties may be identical or at least have a similar structure, i.e. may merely differ in respect of one or a few residues. Using two or more nuclear localization moieties significantly improves the transfer of nucleic acids into the nucleus and therefore leads to an increased transfection efficiency.

The present invention further concerns a composition for transfecting eukaryotic cells, said composition comprising the transport agent according to the invention and at least one cationic polymer and/or cationic lipid capable of transporting the resulting complex from outside of the cell into the cytoplasm.

The invention also relates to a pharmaceutical composition comprising the transport agent according to the invention and common adjuvants and/or carrier substances.

The problem underlying the invention is further solved by a method for producing a transport agent for transporting nucleic acids into eukaryotic cells, said method comprising the steps of
a) providing a complex forming moiety that is capable of forming complexes with at least one nucleic acid molecule and condensing said nucleic acid molecule,
b) providing a nuclear localization moiety comprising at least one nuclear localization signal having a positively charged core sequence that is capable of mediating nuclear transport,
c) neutralizing at least partially the positive net charge of said core sequence by coupling to said core sequence at least one charge carrier having a negative net charge in order to achieve an approximately neutral net charge of said nuclear localization moiety, and
d) linking said complex forming moiety to said nuclear localization moiety directly or via a spacer comprising, e.g., of further (neutral) amino acids, 2-aminoethoxy-2-ethoxy acetic acid (AEEA) or polyethylene glycol (PEG). The moieties may be linked by covalent bonds, hydrogen bonds or ionic interactions.

In an alternative embodiment of the above method, in step a) the complex forming moiety may already be provided in complex with a nucleic acid to be transfected.

The method according to the invention can be advantageously used for producing the transport agent according to the invention as described above.

The transport agent(s) according to the invention can be advantageously used in a method for transporting nucleic acids into eukaryotic cells, wherein a nucleic acid to be transported is incubated with a transport agent according to the invention in order to form a complex comprising said nucleic acid and said transport agent, and incubating said complex with at least one eukaryotic cell. Alternatively, the nucleic acid to be transported can at first be incubated with the complex forming moiety of the transport agent which is then coupled to the nuclear transport moiety before incubating the resulting complex with the cells.

The transport agent(s) according to the invention can further be advantageously used in a method for transporting nucleic acids into the nucleus of eukaryotic cells, wherein a nucleic acid to be transported is incubated with a transport agent according to the invention in order to form a complex of said nucleic acid and said transport agent and in addition with at least one cationic polymer and/or cationic lipid capable of transporting said complex from outside the cell into the cytoplasm, and incubating the resulting complex with at least one eukaryotic cell. Alternatively, the nucleic acid to be transported can at first be incubated with the complex forming moiety of the transport agent which is then coupled to the nuclear transport moiety before incubating the resulting complex with the cationic polymer and/or cationic lipid.

In a preferred embodiment of the invention, the above method(s) are accomplished with eukaryotic cells that are resting, slowly dividing or non-dividing cells, preferably primary cells and or at least one DNA molecule as nucleic acid.

Various and preferred embodiments of the invention are described below in detail.

### Examples

### Peptide mediated electrophoretic mobility shifts of DNA upon binding to peptide

DNA interacting unspecifically with peptides through positively charged amino acids may mask positively charged nuclear localisation sequences (NLS) and interfere with their function in nuclear transport. In order to demonstrate that peptides with a positive net charge form higher molecular weight complexes with DNA, peptides bearing an NLS and additional negatively charged amino acids were tested for forming complexes with DNA.

### Experimental details

Peptides were synthesized by F-moc-Chemistry and HPLC-purified to 95% purity. The carboxyl-group at the C-terminus was amidated and the N-terminal amino-group was actylated, in order to eliminate additional charges.

Sequences are the following:

### NLS-1

GSGSPKKKRKVGSG (5-fold positively charged, SEQ ID NO:1)
SV40 large T-antigen core NLS (PKKKRKV) with arbitrarily chosen flanking amino acids (glycine and serine)

### NLS-2

EEDTPPKKKRKVED (no net charge, SEQ ID NO:2)
SV40 large T-antigen core NLS (PPKKKRKV) with EED N-terminal flanking region of the NLS form Polyoma virus VP2-protein

### NLS-3

MASQGTKRSYEQMETDGERQYC (1-fold negatively charged, SEQ ID NO:3)
NLS of the Influenza virus nuclear protein

### SV21

GKPTADDQHSTPPKKKRKVED (2-fold positively charged, SEQ ID NO:4)
Mutated part of the SV40 large T-antigen bearing the core NLS

### SV27

GKPSSDDEATADSQHSTPPKKKRKVED (no net charge, SEQ ID NO:5)
Natural part of the SV40 large T-antigen bearing the core NLS

Lyophilized peptides were solved in distilled water to 1mg/ml.

DNA was pmaxGFP plasmid DNA (4.4 kb size).

1µg DNA was incubated with peptide (µg amounts as indicated in the figure) in water in a total volume of 20µl at 20°C for 30 min. After that loading dye was added, each sample filled into a slot of a 0.6% agarose gel (Tris-acetate/EDTA as running buffer, pH 8.5). Electrophoresis was run at 90V for 120min. P = 1µg plasmid DNA without peptide. M = kb marker (bands indicating 1.5, 2, 3, 4, 5, and 6 kb). The gel was stained with Ethidiumbromide for 10 min, than documented using a BioRad Gel-Doc System.

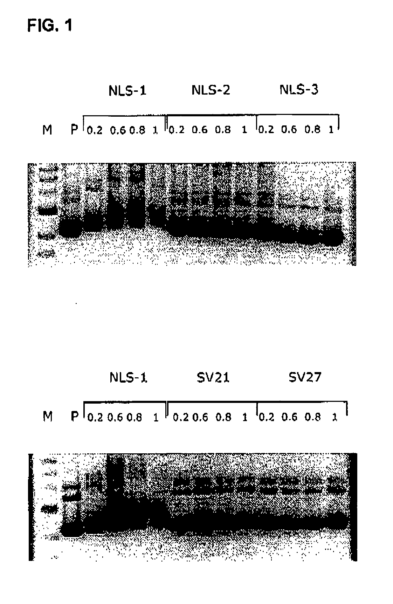

The NLS-1 peptide due to its 5-fold positive charge does bind to DNA and forms aggregates, therefore altering the electrophoretic mobility of DNA, causing a shift in apparent molecular weight (bands are running at higher molecular weight). All other peptides (of neutral charge, with one negative net charge or a 2-fold positive net charge) do not cause any alteration of the electrophoretic mobility of the DNA even at higher amounts of peptide (**FIG. 1**). The NLS on the NLS-1 peptide may be masked and altered in its capacity to mediate nuclear translocation, whereas all other peptides are still functioning in nuclear import.

### Peptide mediated electrophoretic mobility shifts of DNA upon binding to peptide

DNA interacting unspecifically with peptides through positively charged amino acids may mask positively charged nuclear localisation sequences (NLS) and interfere with their function in nuclear transport. In order to demonstrate what positive net charge on a peptide is necessary to form higher molecular weight complexes with DNA, peptides bearing an NLS and additional negatively charged amino acids were tested for forming complexes with DNA. Peptides were designed with different positive net charges, 5+, 4+, 3+, 2+, 0±, by exchanging neutral amino acids for acidic ones or vice versa acidic for neutral amino acids

### Experimental details

Peptides were synthesized by F-moc-chemistry and HPLC-purified to 95% purity. The carboxyl-group at the C-terminus was amidated and the N-terminal amino-group was actylated, in order to eliminate additional charges.

Sequences are the following:

### NLS1(5+) (=NLS-1, see above)

GSGSPKKKRKVGSG (5-fold positively charged, SEQ ID NO:1)
SV40 large T-antigen core NLS (PKKKRKV) with arbitrarily chosen flanking amino acids (glycine and serine)

### NLS1(4+)c

ESGSPKKKRKVGSG (4-fold positively charged, SEQ ID NO:6)

### NLS1(4+)d

GSGSPKKKRKVDSG (4-fold positively charged, SEQ ID NO:7)

### NLS1(3+)c

ESGSPKKKRKVDSG (3-fold positively charged, SEQ ID NO:8)

### NLS1(3+)d

EDGSPKKKRKVGSG (3-fold positively charged, SEQ ID NO:9)

### NLS1(3+)e

GSGEPKKKRKVDSG (3-fold positively charged, SEQ ID NO:10)

### NLS2(0±) (=NLS-2, see above)

EEDTPPKKKRKVED (no net charge, SEQ ID NO:2)
SV40 large T-antigen core NLS (PPKKKRKV) with EED N-terminal flanking region of the NLS form Polyoma virus VP2-protein

### NLS2(3+)a

GSDTPPKKKRKVES (3-fold positively charged, SEQ ID NO:11)

### NLS2(3+)b

GSGTPPKKKRKVED (3-fold positively charged, SEQ ID NO:12)

### NLS2(4+)a

GESTPPKKKRKVGS (4-fold positively charged, SEQ ID NO:13)

### NLS2(4+)b

GSGTPPKKKRKVES (4-fold positively charged, SEQ ID NO:14)

### SV21(2+) (=SV21, see above)

GKPTADDQHSTPPKKKRKVED (2-fold positively charged, SEQ ID NO:4)
Mutated part of the SV40 large T-antigen bearing the core NLS

### SV21(3+)e

GKPTAGDQHSTPPKKKRKVED (3-fold positively charged, SEQ ID NO:15)

### SV21(4+)e

GKPTADDQHSTPPKKKRKVSG (4-fold positively charged, SEQ ID NO:16)

Lyophilized peptides were solved in distilled water to 0.1 mM.

DNA was pmaxGFP plasmid DNA (4.4 kb size).

1µg DNA was incubated with 1nM peptide in water in a total volume of 20µl at 20°C for 30 min. After that loading dye was added, each sample filled into a slot of a 0.6% agarose gel (Tris-acetate/EDTA as running buffer, pH 8.5). Electrophoresis was run at 90V for 120min. P = 1µg plasmid DNA without peptide. M = kb marker (bands indicating 1.5, 2, 3, 4, 5, and 6 kb). The gel was stained with Ethidiumbromide for 10 min, than documented using a BioRad Gel-Doc System.

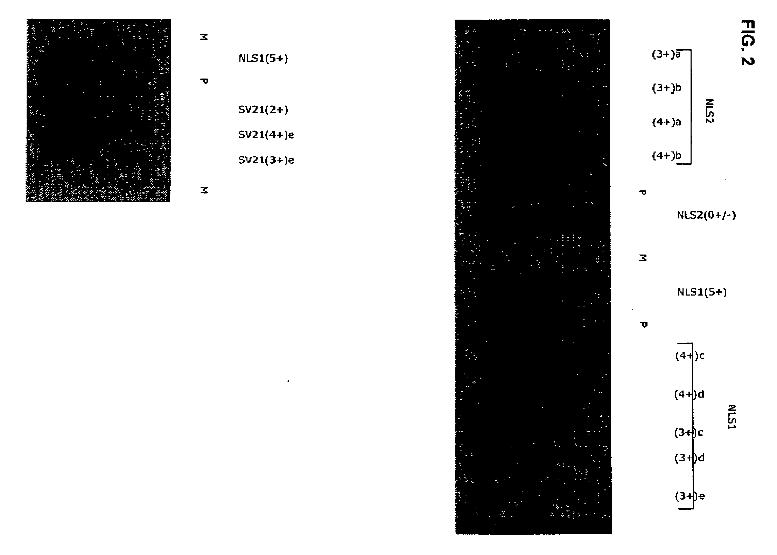

The NLS-1 peptide due to its 5-fold positive charge and the peptides with a 4-fold positive net charge do bind to DNA and form aggregates, therefore altering the electrophoretic mobility of DNA, causing a shift in apparent molecular weight (bands are running at higher molecular weight). All other peptides (of neutral charge or with a 3-fold positive net charge) do not cause any alteration of the electrophoretic mobility of the DNA even at higher amounts of peptide (**FIG. 2**). The NLS on the NLS-1 peptide may be masked and altered in its capacity to mediate nuclear translocation, whereas all other peptides are still functioning in nuclear import.

### Literature

Boulikas, T. (1993). Nuclear localization signals (NLS). Crit Rev Eukaryot Gene Expr 3, 193-227.

Emi, N. et al. (1997). Gene transfer mediated by polyarginine requires a formation of big carrier-complex of DNA aggregate. Biochem Biophys Res Comm 231, 421-424.

Görlich, D. (1998). Transport into and out of the cell nucleus. EMBO J 17, 2721.

Nagasaki, T. et al. (2003). Can nuclear localization signals enhance nuclear localization of plasmid DNA. Bioconjug Chem 14, 282-286.

Neumann, G. et al. (1997). Nuclear import and export of influenza virus nucleoprotein. J Virol 71, 9690-9700.

Subramanian, A. et al. (1999). Nuclear targeting peptide scaffolds for lipofection of nondividing mammalian cells. Nature Biotechnology 17, 873-877.

Wang, P. et al. (1997). The NPI-1/NPI-3 binding site on the influenza a virus nucleoprotein NP is a nonconventional nuclear localization signal. J Virol 71, 1850-1856.

Yoneda, Y. et al. (1987). Synthetic peptides containing a region of SV 40 large T-antigen involved in nuclear localization direct the transport of proteins into the nucleus. Exp cell Res 170, 439-453.

Yoneda, Y. et al. (1992). A long synthetic peptide containing a nuclear localization signal and its flanking sequences of SV 40 T-antigen directs the transport of IgM into the nucleus effectively. Exp cell Res 201, 313.

## Claims

1. A transport agent for transporting nucleic acids into eukaryotic cells, said transport agent comprising a complex forming moiety that is capable of forming complexes with at least one nucleic acid molecule and condensing said nucleic acid molecule, and at least one nuclear localization moiety comprising at least one nuclear localization signal and having an approximately neutral net charge.

2. The transport agent according to claim 1, wherein said complex forming moiety comprises a basic peptide, a basic polypeptide or a basic protein.

3. The transport agent according to claim 1 or 2, wherein said complex forming moiety comprises a cationic peptide, a cationic lipid or a non-lipid cationic polymer.

4. The transport agent according to claim 1, 2 or 3, wherein said complex forming moiety comprises Polylysine, Polyarginine, Polyornithine or Polyhistidine and/or any polypeptide composed of any mixture of the amino acids Lysine, Arginine, Ornithine and Histidine.

5. The transport agent according to claim 1, 2 or 3, wherein said complex forming moiety comprises a protein belonging to the High Mobility Group protein or Histone family.

6. The transport agent according to any one of claims 1 to 5, wherein said nuclear localization signal comprises a core sequence that is capable of mediating transport of said nucleic acid into the nucleus of a cell, said core sequence having a positive net charge.

7. The transport agent according to any one of claims 1 to 6, wherein said nuclear localization moiety further comprises at least one charge carrier that has a negative net charge.

8. The transport agent according to claim 7, wherein said charge carrier comprises at least one negatively charged amino acid.

9. The transport agent according to claims 6, 7 or 8, wherein the approximately neutral net charge of said nuclear localization moiety is achieved by at least partially neutralizing the positive net charge of the core sequence with said at least one charge carrier.

10. The transport agent according to any one of claims 1 to 9, wherein said nuclear localization moiety has at most a 3-fold positive net charge.

11. The transport agent according to any one of claims 1 to 10, wherein said nuclear localization signal comprises a native amino acid sequence that has a positive net charge.

12. The transport agent according to any one of claims 1 to 11, wherein said nuclear localization moiety comprises at least one bipartite nuclear localization signal.

13. The transport agent according to any one of claims 1 to 12, wherein said nuclear localization moiety comprises at least one amino acid sequence according to SEQ ID NO:2, SEQ ID NO:3, SEQ ID NO:4, SEQ ID NO:5, SEQ ID NO:8, SEQ ID NO:9, SEQ ID NO:10, SEQ ID NO:11, SEQ ID NO:12 and/or SEQ ID NO:15.

14. The transport agent according to any one of claims 1 to 13, comprising two or more nuclear localization moieties.

15. A composition for transfecting eukaryotic cells, said composition comprising the transport agent according to any one of claims 1, 2 or 4 to 14 and at least one cationic polymer and/or cationic lipid capable of transporting the resulting complex from outside of the cell into the cytoplasm.

16. A pharmaceutical composition comprising the transport agent according to any one of claims 1 to 14 and common adjuvants and/or carrier substances.

17. A method for producing a transport agent for transporting nucleic acids into eukaryotic cells, said method comprising the steps of
a) providing a complex forming moiety that is capable of forming complexes with at least one nucleic acid molecule and condensing said nucleic acid molecule,
b) providing a nuclear localization moiety comprising at least one nuclear localization signal having a positively charged core sequence that is capable of mediating nuclear transport,
c) neutralizing at least partially the positive net charge of said core sequence by coupling to said core sequence at least one charge carrier having a negative net charge in order to achieve an approximately neutral net charge of said nuclear localization moiety, and
d) linking said complex forming moiety to said nuclear localization moiety directly or via a spacer.

18. The method according to claim 17 for producing the transport agent according to any one of claims 1 to 14.

19. A method for transporting nucleic acids into eukaryotic cells, wherein a nucleic acid to be transported is incubated with a transport agent according to any one of claims 1 to 14 in order to form a complex comprising said nucleic acid and said transport agent, and incubating said complex with at least one eukaryotic cell.

20. A method for transporting nucleic acids into the nucleus of eukaryotic cells, wherein a nucleic acid to be transported is incubated with a transport agent according to any one of claims 1, 2 or 4 to 14 in order to form a complex of said nucleic acid and said transport agent and in addition with at least one cationic polymer and/or cationic lipid capable of transporting said complex from outside the cell into the cytoplasm, and incubating the resulting complex with at least one eukaryotic cell.

21. The method according to claim 19 or 20, wherein said eukaryotic cells are resting, slowly dividing cells or non-dividing cells, preferably primary cells.

22. The method according to claim 19, 20 or 21, wherein said nucleic acid is at least one DNA molecule.
